# FASCICULE DE BREVET EUROPEEN

(11) **EP 0 386 187 B1**
(45) Date de publication et mention de la délivrance du brevet: **14.09.1994**
(21) Numéro de dépôt: 89908203.6
(22) Date de dépôt: 29.06.1989
(51) Int. Cl.: C07C 43/13, C07C 215/08, C07C 217/08, C07C 233/18, C07C 233/36, C07C 311/03, C07C 321/14, C07D 303/24, C07D 303/34, C07D 303/36

(54) **NOUVEAUX COMPOSES FLUORES REACTIFS, HYDROSOLUBLES; LEURS OBTENTIONS ET LEURS UTILISATIONS**
REAKTIVE WASSERLÖSLICHE FLUORIERTE VERBINDUNGEN, IHRE HERSTELLUNG UND VERWENDUNG
NEW WATER-SOLUBLE REACTIVE FLUORINE-CONTAINING COMPOUNDS; METHODS FOR OBTAINING THEM AND UTILIZATIONS

(30) Priorité: 24.08.1988 FR 8811345
(43) Date de publication de la demande: 12.09.1990
(73) Titulaire: SZONYI, Istvan, F-06300 Nice (FR); SZONYI, Stéphane, MC-98000 Monte Carlo (MC); SZONYI, François, MC-98000 Monte Carlo (MC)
(72) Inventeur: SZONYI, Istvan, F-06300 Nice (FR); SZONYI, Stéphane, MC-98000 Monte Carlo (MC); SZONYI, François, MC-98000 Monte Carlo (MC)
(86) Numéro de dépôt international: FR8900338
(87) Numéro de publication internationale: WO9002110

(56) Documents cités:
- EP-A- 0 125 826
- DE-A- 2 052 579
- DE-A- 2 350 513
- US-A- 3 976 698
- US-A- 3 980 715
- PATENT ABSTRACTS OF JAPAN, vol. 6, Nr. 154 (C-119)(1032), 14 August 1982; & JP-A-5772977

## Description

La présente invention concerne de nouveaux composés hautement fluorés, hydrosolubles, contenant une fonction réactive, de type halohydrine ou époxyde, éloignée de la chaînes fluorée.

On connait quelques travaux interessants portant sur les composés fluorés de type halohydrine et époxyde comme par exemple la demande de brevet francais n° 2529890 qui décrit les deux produits principaux suivants : 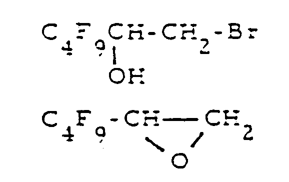
Ces composés sont réputés avoir une bonne réactivité avec les produits possédant un hydrogène mobile. Leurs réactions conduisent à l'obtention de tensioactifs fluorés, qui sont décrits dans la demande de brevet français n° 2530623.

Malheureusement ces époxydes et ces halohydrines sont insolubles dans l eau, étant donné la proximité immédiate de la chaîne perfluorée dénuée de radical solubilisant.

Les brevets japonais n° 55-162751, 57-72977 et 61-71830 mentionnent les composés suivants : 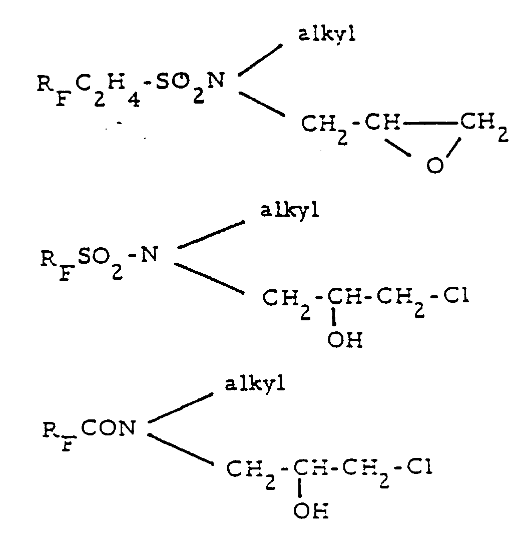
Le brevet européen n° 0125826 propose les produits suivants : 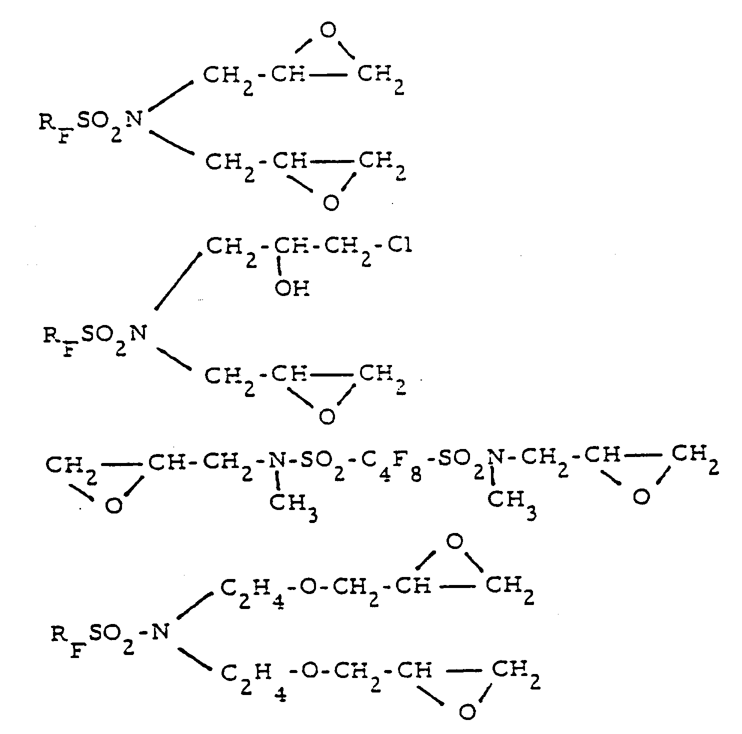
Ces composés bifonctionnels sont destinés à la fabrication de résine par polymérisation avec des produits adéquats.

Dans toutes les molécules citées ci-dessus, la fonction époxyde ou chlorhydrine est située près de la chaîne perfluorée ; ces produits sont insolubles dans l'eau.

Le but de la recherche a donc été de synthétiser des composés fluoroalkylés ayant des groupements réactifs époxyde ou halohydrine, éloignés de la chaîne fluorée, afin d'obtenir des produits dispersibles ou solubles dans l'eau.

Maintenant il a été trouvé et c'est ce qui constitue l'objet de la présente invention, de nouveaux composés perfluroalkylés réactifs, répondant à la formule générale suivante : 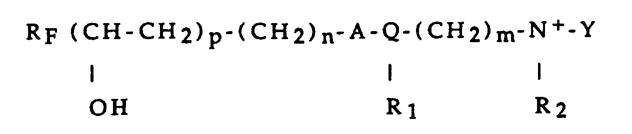
R_{F} : est un perfluoro ou polyfluoroalkyle linéaire ou ramifié, contenant 4 à 20 atomes de carbone.
p : est 0 ou 1
n : est un nombre entier de 0 à 3
m : est un nombre entier de 3 à 6
A : est un radical -SO₂-, ou une simple liaison 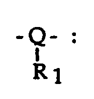
est imino ou imino substitué par alkyl, oxygène ou soufre
R₂ : deux méthyles ou deux éthyles.
Y : est un radical : 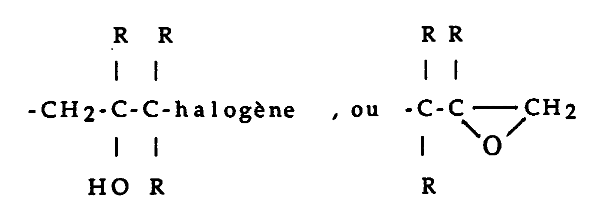
où R est un atome d'hydrogène, ou un radical méthyle ou éthyle
Ces produits sont dispersibles ou solubles dans l'eau et conservent leur forme réactive en solution aqueuse.

Pour la préparation des nouveaux composés selon l'invention, on a plusieurs possibilités. Par exemple les fluorosulfonamides contenant une amine tertiaire, proposés par le brevet US 2759019, peuvent constituer la matière première de départ pour la fabrication des fluoro-composés réactifs selon l'invention. La réaction en milieu acide ou en milieu neutre s'effectue selon le schéma suivant : 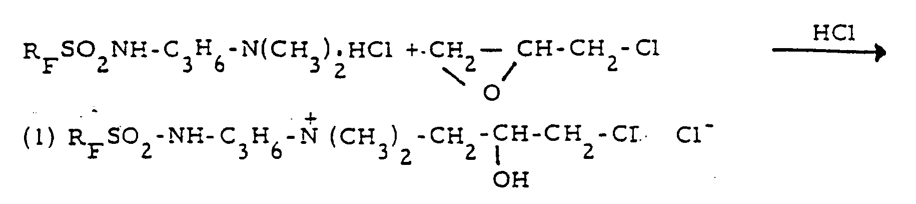
On peut utiliser pour les synthèses selon l'invention l'épichlorhydrine du glycérol que l'on trouve facilement dans le commerce mais également plusieurs autres épichlorhydrines , comme par exemple : 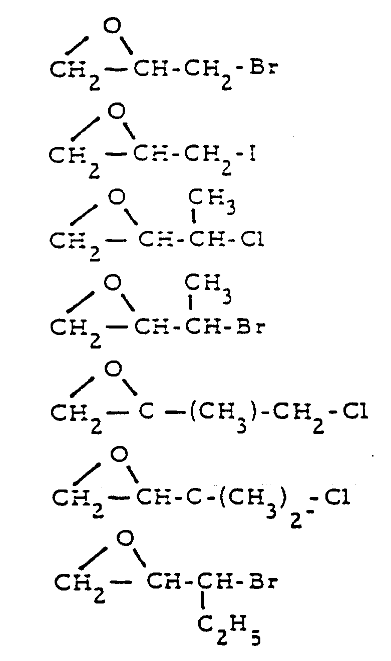
L'ammonium quaternaire obtenu (1) est parfaitement soluble dans l'eau et garde son état réactif sans décomposition en solution aqueuse à la température ambiante et à pH neutre.

D'autres matières premières de départ peuvent servir à l'obtention des composés fluorés réactifs selon l'invention ; on peut en indiquer quelques unes sans toutefois en épuiser le nombre dans les réactions suivantes : 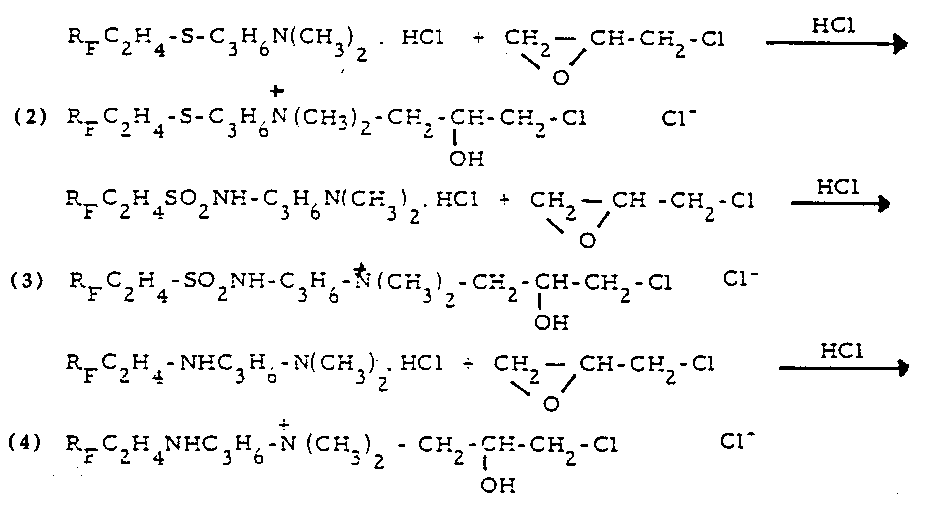
Les produits obtenus,conformes à l'invention sont aptes à réagir avec les amines, les alcools, les thiols, et les acides gras pour donner des composés fluorés stables. Par exemple avec la diéthanolamine, on a le schéma suivant : 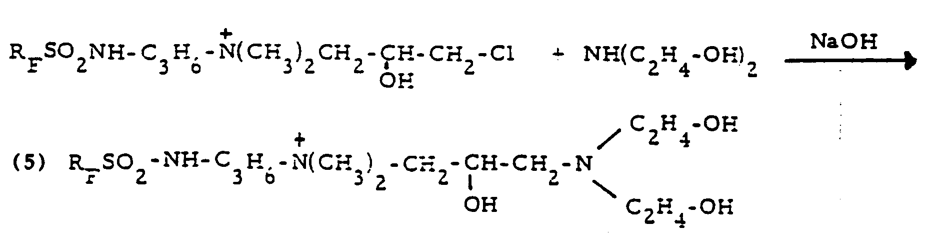
Les composés selon l'invention réagissent facilement avec les amines et les thiols et par conséquent, on peut les utiliser en tant que fluoroalkylants des hydrolysats de proteine, selon le schéma suivant : 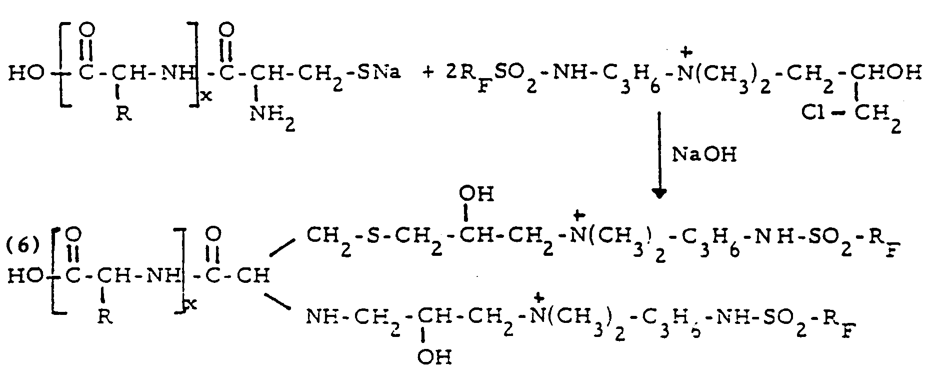
Les produits selon l'invention réagissent également avec les poly - amines.

Les composés conformes à l'invention produisent des tensioactifs fluorés avec les proteines aminolysées.

Avec les alcools gras les polyols et les polysaccharides, les composés conformes à l'invention réalisent d'excellents émulseurs.

Avec les polyglycols la réaction est la suivante : 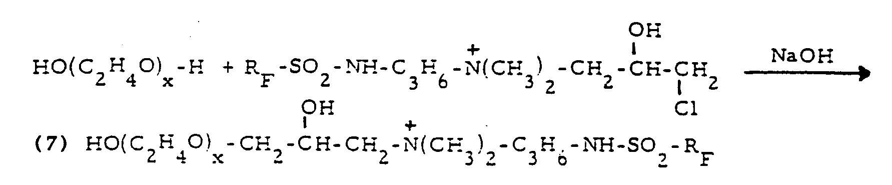
Avec des acides carboxyliques les produits selon l'invention peuvent donner des esters selon le schéma suivant : 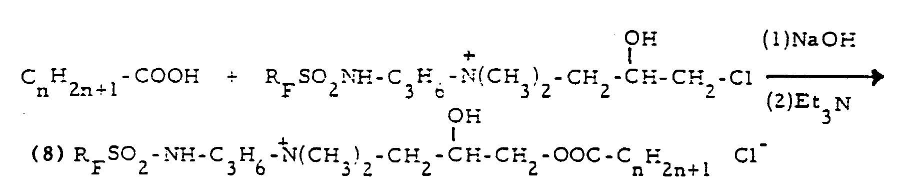
Les composés selon l'invention peuvent par hydrolyse conduire à des fluoroglycols.

En milieu basique, la polymérisation est facilitée pour certains produits selon l'invention, par exemple : 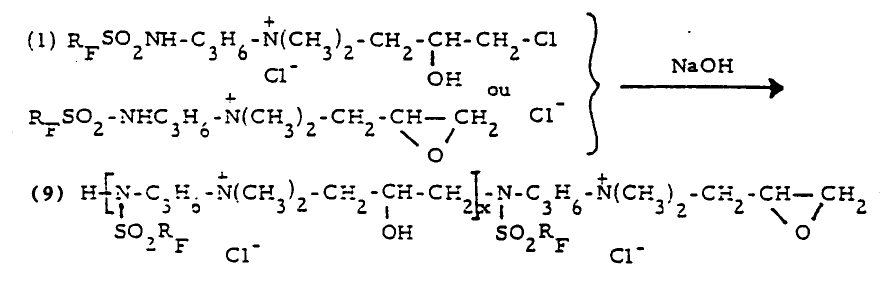
En présence d'un excés de DMAPA et de l'épichlorhydrine du glycérol lors de la préparation du produit(1) on a la possibilité d'effectuer une copolymérisation selon le schéma suivant : 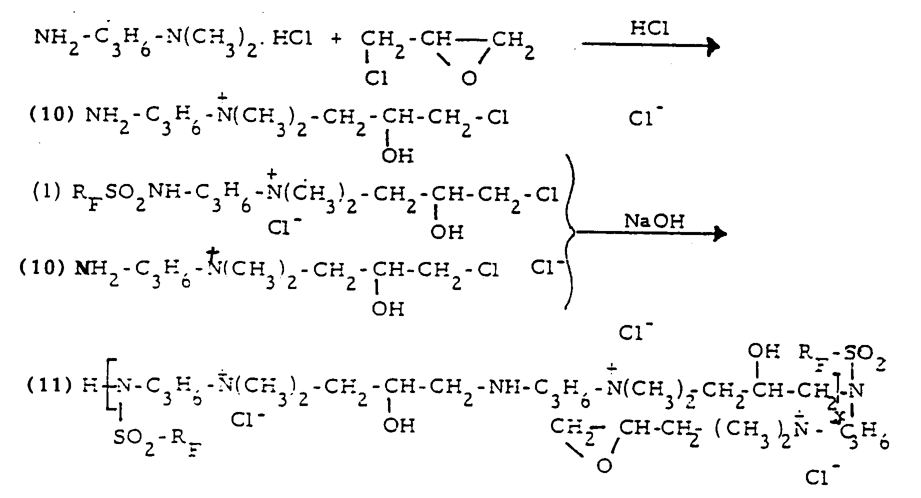
A partir de la sulfonamide utilisée pour l'obtension du produit(1), et de l'amine en excès, les réactions en milieu basique sont les suivantes : 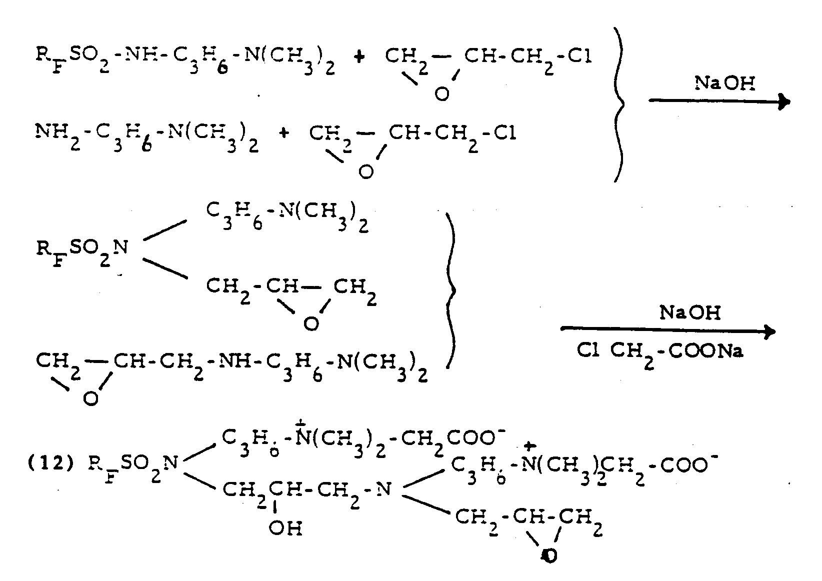
Nous pouvons obtenir des macromolécules avec les composés selon l'invention, par exemple : Le produit obtenu par action de diethanolamine (5) peut réagir avec le copolymère de méthoxyvinyl anhydride maleique, selon la réaction suivante : 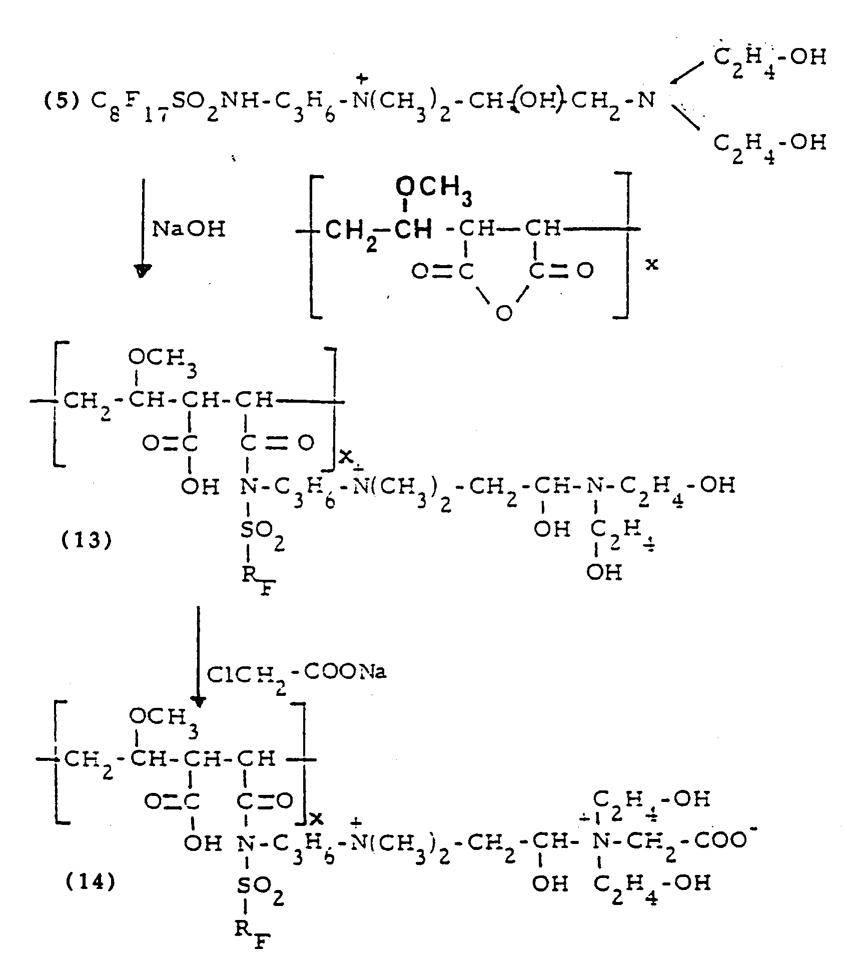
Les nouveaux composés fluorés selon l'invention peuvent être utilisés en général comme agents fluoroalkylants dans les techniques d'oléophobie et d'hydrophobie et en particulier dans la fluoro-alkylation des dérivés de proteines, des polyols ou des polysaccharides.

Cependant ils sont utilisables en tant que tensioactifs fluorés pour abaisser la tension superficielle de l'eau et en tant qu'agents mouillant et dispersant ; ils peuvent être appliqués dans la préparation de compositions bactéricides et d'inhibiteurs de corrosion, ainsi que dans la fabrication d'agents extincteurs.

Les exemples suivants illustrent l'invention et montrent comment celle-ci peut être mise en pratique, toutefois ces exemples ne sont nullement limitatifs.

### Exemple 1

Dans un ballon muni d'un agitateur et d'un réfrigérateur à reflux, on introduit 15 g (0,15 mole) de dimethyl aminopropylamine dilué dans 10 g de diméthylformamide.

On ajoute goutte à goutte 25 g (0,05 mole) de fluorure de perfluoro - octylsulfonyle en agitant à la température de 80°C. Après l'addition, on maintient la température à 80°C en agitant encore 2 heures.

On laisse refroidir à 40°C et on verse 15 ml d'acide chlorhydrique à 32%, dilué dans 100ml d'eau. On mélange pendant 30 minutes pour obtenir une dissolution parfaite. On verse lentement, en plusieurs portions, en 15 minutes environ, 14 g (0,15 mole) de l'épichlorhydrine du glycérol. On continue de mélanger à 40-45°C pendant une heure et ensuite à 50-60°C pendant cinq heures.

La solution obtenue pèse 181 g, et contient environ 30% de matière active. Elle se présente sous l'aspect d'un liquide sirupeux, se dissout parfaitement dans l'eau, sa densité est d'environ 1,135 à 20°C et son pH 6,5 à 7.
Résultats des mesures de tension superficielle :

| Solution de la matière brute en % | Tension superficielle dynes/cm |
|---|---|
| 0,1 | 16,3 |
| 0,01 | 18,9 |
| 0,001 | 35 |

### Exemple 2

On verse 100 g de proteine hydrolysée (matière active 25%) dans un ballon muni d'un agitateur et d'un réfrigérant à reflux.

On ajoute de la soude caustique en solution concentrée pour régler l'alcalinité du milieu afin que l'on obtienne le virage à la couleur rose de la phenolphtaleine, et on introduit en mélangeant 1g du produit obtenu selon l'exemple 1. On chauffe pendant deux heures à 80°C en agitant. Ensuite on laisse refroidir et on neutralise avec l'acide chlorhydrique à pH7-7,5.
Résultats des mesures de tension superficielle :

| solution de la matière brute en % | Tension superficielle dynes/cm | Tension interfaciale |
|---|---|---|
| 6 | 24 | 2,6 |
| 3 | 26,7 | 3,8 |

### Exemple 3

On introduit dans un ballon 18 g de produit obtenu selon l'exemple 1. On verse 1 g (0.01 mole) de diéthanolamine et 0,8 g (0,02 mole) de soude caustique dissout dans 1cm³ d'eau. On chauffe le mélange pendant trois heures à 80°C. En suite on verse le liquide obtenu dans une solution de 7,6 g (0,05 mole) de copolymère de métoxyvinyle/anhydride maleique dissout dans 18 g de diméthylformamide. On chauffe en agitant pendant douze heures à 110°C à reflux.

Le poids du produit obtenu est d'environ 45 g (30% de matière active) et se présente sous forme d'un liquide épais qui servira pour obtenir un tensioactif (ex. 4) ou réaliser un condensat avec une proteine hydrolysée (ex. 5)

### Exemple 4

On pèse dans un ballon 45 g de produit visqueux obtenu selon l'exemple 3. On ajoute 1,75 g (0,015 mole) de monochloracétate de soude dispersé dans 3 g de diméthylformamide ; on maintient le mélange à 80°C en agitant pendant 6 heures. Ensuite on ajoute 5 g de potasse caustique dissout dans 10,3 ml d'eau et on mélange pendant six heures à 70-80°C.

On obtient environ 65 g de produit (20-22% de matière active) parfaitement soluble dans l'eau.
Résultat des mesures de tension superficielle :

| Solution de la matière brute en % | Tension superficielle en dynes/cm |
|---|---|
| 0,1 | 16,5 |
| 0,01 | 17,2 |
| 0,001 | 42 |

### Exemple 5

On verse dans un ballon 45 g de produit obtenu selon l'exemple 3 et on ajoute 1,84 g (0,015 mole) de monochloracétate d'éthyle et on mélange à la température de 80°C pendant six heures. Ensuite, on introduit 100 g de proteine hydrolysée contenant 25% de polypeptides et 10% de potasse caustique.

On chauffe le mélange pendant quatre heures (à reflux) à 70-80°C. On neutralise par addition d'acide chlorhydrique à pH 7,'5-8. La solution obtenue pèse 150 g et contient environ 26% de matière active.
Résultats des mesures de tension superficielle :

| solution de la matière brute en % | Tension superficielle en dynes/cm |
|---|---|
| 0,1 | 18 |
| 0,01 | 20 |
| 0,001 | 50 |

### Exemple 6:

Dans un ballon de 500 ml, on introduit 20 g de corne et de sabot broyés, 20 g de diethanolamne et 20 g de monoethylène glycol. On chauffe une heure à 100°C pour enlever l'humidité. Ensuite on augmente progressivement la température et on mélange la masse réactionnelle pendant cinq heures à 160°C à reflux. Après avoir abaissé la température à 80°C, on ajoute 140 g de produit obtenu selon l'exemple 1. On chauffe le mélange pendant cinq heures à la température de 80°C. A la fin de la réaction, on ajoute 150 ml d'eau et on filtre. Le liquide obtenu content environ 32% de matière active.
Résultats des mesures de tension superficielle :

| solution de la matière brute en % | Tension superficielle en dynes/cm | Tension interfaciale |
|---|---|---|
| 0,1 | 16 | 3,5 |
| 0,01 | 18 | |
| 0,001 | 30 | |

### Exemple 7 :

On verse 40 g de produit obtenu selon l'exemple 1 dans un ballon muni d'un réfrigérant à reflux et d'un agitateur. On ajoute 2 à 3 ml de potasse caustique à 50% et on chauffe le mélange fortement basique pendant huit heures à la température de 80°C. On obtient un fluide visqueux ayant environ 30% de matière active.
Résultats de mesures de tension superficielle :

| Solution de matière brute en % | Tension superficielle en dynes/cm |
|---|---|
| 0,1 | 20,5 |
| 0,01 | 35,3 |
| 0,001 | 53,8 |

### Exemple 8

On dispose 170g de produit obtenu selon l'exemple 9 dans un ballon et on verse 40 g (0,1 mole) de polyéthylène glycol (masse moléculaire 400) en mélangeant. On vérifie le pH et éventuellement on le réajuste au pH 11 à l'aide d'une solution de soude caustique à 45%. On chauffe à 80°C en agitant pendant cinq heures à reflux.

Le produit obtenu qui pèse environ 210 g est une solution de polyglycol perfluoroalkylé, soluble dans l'eau en toute proportion (environ 40% de matière active).
Résultats des mesures de tension superficielle :

| Solution de la matière brute en % | Tension superficielle en dynes/cm |
|---|---|
| 0,1 | 21 |
| 0,01 | 23 |
| 0,001 | 40 |

### Exemple 9:

On introduit dans un ballon muni d'un agitateur 48 g (0,1 mole) du produit C₆F₁₃-CH(OH)CH₂-S-C₃H₆-N(CH₃)₂ obtenu selon la demande de brevet français n°2530623 et on verse 11ml d'acide chlorhydrique à 30% dilué préalablement dans 100 ml d'eau. Après dissolution, on ajoute lentement 9,3 g (0,1 mole) de l'épichlorhydrine du glycerol à 40°C et on continue de mélanger à 40-50°C pendant quatre heures.

La solution d'alpha chlorhydrine perfluoroalkylé obtenue pèse environ 170 g, a un pH de 6,5 à 7, et est parfaitement soluble dans l'eau (matière active environ 30%).
Résultats des mesures de tension superficielle :

| Solution de la matière brute en % | Tension superficielle en dynes/cm |
|---|---|
| 0,1 | 17 |
| 0,01 | 19,2 |
| 0,001 | 35 |

### Exemple 10

On dispose 50 g de solution obtenue selon l'exemple 9 et on ajoute 20ml d'acide chlorhydrique à 32%. On chauffe le mélange jusqu'à ébullition pendant deux heures. La solution obtenue pèse environ 70 g et contient environ 20-22% de produit actif (triol perfluoroalkyle).
Résultats des mesures de tension superficielle :

| Solution de la matière brute en % | Tension superficielle en dynes/cm |
|---|---|
| 0,1 | 17,2 |
| 0,01 | 20 |
| 0,001 | 40 |

## Revendications

1. Nouveaux composés perfluoroalkylés réactifs, hydrosolubles ou dispersibles dans l'eau, caractérisés par le fait qu'ils contiennent un seul groupement réactif, époxyde ou halohydrine, éloigné du radical perfluoré, et que la molécule contient au moins un atome d'azote quaternaire organique, près du groupement réactif, répondant à la formule générale suivante : 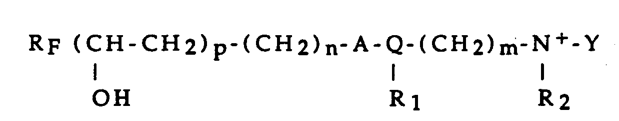 R_{F} : est un perfluoro ou polyfluoroalkyle linéaire ou ramifié, contenant 4 à 20 atomes de carbone.
p : est 0 ou 1
n : est un nombre entier de 0 à 3
m : est un nombre entier de 3 à 6
A : est un radical -SO₂-, ou une simple liaison 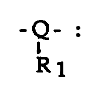 est imino ou imino substitué par alkyl, oxygène ou soufre
R₂ : diméthyle ou diéthyle.
Y : est un radical : 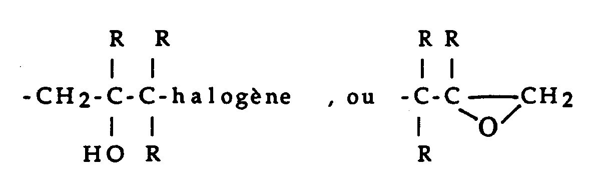 où R est un atome d'hydrogène, ou un radical méthyle ou éthyle.

2. Procédés d'obtention de nouveaux composés perfluoroalkylés selon la revendication (1), caractérisés par le fait qu'on fait réagir une épihalohydrine sur un composé perfluoro ou polyfluoroalkylé, ayant une fonction amine tertiaire éloignée de la chaîne fluorée, répondant à la formule générale suivante : 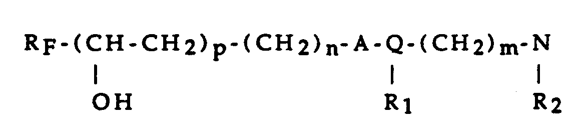 Les significations sont définies dans la revendication (1).

3. Procédés d'obtention de nouveaux composés perfluoroalkylés selon la revendication (2), caractérisés par le fait que l'épihalohydrine est l'épichlorhydrine du glycerol.

4. Nouveaux composés fluorés selon la revendication (1), caractérisés par le fait qu'on les emploie en général comme agents fluoroalkylants dans les techniques d'oléophobie et/ou d'hydrophobie et/ou d'alcophobie et en particulier dans la fluoroalkylation des dérivés de protéines, des polyols ou des polysaccharides.

5. Nouveaux composés fluorés, selon la revendication (1), caractérisés par le fait qu'on les utilise comme agents tensioactifs, mouillants, dispersants et composants pour inhibiteurs de corrosion ou bactéricide, ou qu'on les emploie dans la fabrication d'agents extincteurs.

## Claims

1. New reactive perfluoroalkylated compounds, water soluble or dispersible in water, characterized by the fact they contain a single reactive group, epoxide or halohydrin, distant from the perfluorinated radical, and the molecule contains at least one atom of organic quaternary nitrogen, near the reactive group, corresponding to the following general formula: 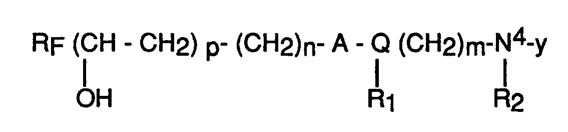 R_{F} : is a linear or ramified perfluoro or polyfluoroalkyl containing 4 to 20 carbon atoms,
A : SO₂ or CO or nothing,
p : is 0 or 1,
n : is an integer from 0 to 3
m : is an integer from 3 to 6,
A : is an -SO₂- radical, or a simple bond, 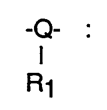 is imino or imino substituted by alkyl, oxygen or sulfur
R₂ : dimethyl or diethyl,
Y : is a radical: 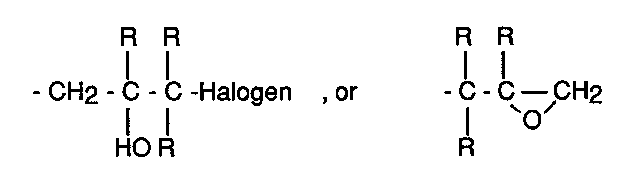 where R is a hydrogen atom, or a methyl or ethyl radical

2. Manufacturing processes for new perfluoroalkyl compounds according to claim (1), characterized by the fact an epihalohydrin is made to react with a perfluoro or polyfluoroalkylated compound with a tertiary amine function distant from the fluorinated chain, corresponding to the following general formula: 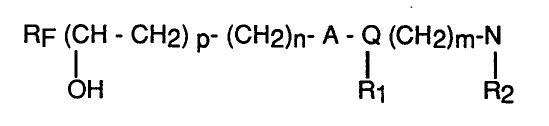 The terms are defined in claim (1).

3. Manufacturing processes for new perfluoroalkyl compounds according to claim (2), characterized by the fact the epihalohydrin is glycerol epichlorhydrin.

4. New fluorinated compounds according to claim (1), characterized by the fact they are generally used as fluoroalkylizing agents in oleophobic and/or hydrophobic and/or alcophobic techniques and in particular in the fluoroalkylation of protein, polyol or polysaccharide derivatives.

5. New fluorinated compounds according to claim (1), characterized by the fact they are generally used as surfactant, wetting, dispersing agents and as components of bactericidal agents or corrosion inhibitors, or in the manufacture of extinguishing agents.

## Patentansprüche

1. Neue reaktive wasserlösliche oder -dispersible Perfluoroalkylen-Verbindungen, dadurch gekennzeichnet, daß sie eine einzige reaktive Gruppe aus Epoxyd oder Halohydrin beinhalten, abgesetzt vom Perfluoro-Radikal, und daß das Molekül mindestens ein quaternäres organisches Stickstoff-Atom nahe der reaktiven Gruppe besitzt, und die folgender allgemeingültiger Formel entspricht: 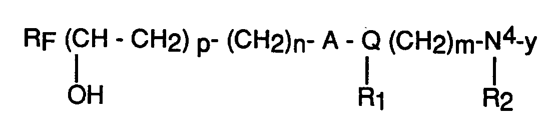 R_{F}; eine lineare oder verzweigte Perfluoro- oder Polyfluoroalkyl-Gruppe mit 4 bis 20 Kohlenstoffatomen
p : 0 oder 1
n : eine ganze Zahl zwischen 0 und 3
m : eine ganze Zahl zwischen 3 und 6
A : ein Radikal - SO₂ - oder eine einfache Verbindung 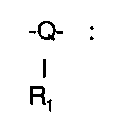 eine Iminogruppe oder eine Iminogruppe ersetzt durch Alkyl, Sauerstoff oder Schwefel
R₂ : Dimethyl oder Diethyl
Y : ein Radikal: 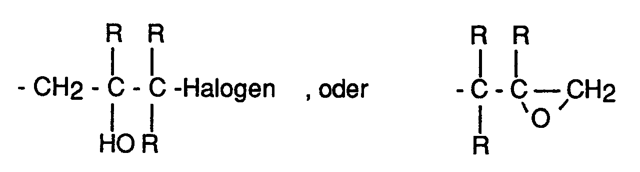 wobei R ein Wasserstoffatom, ein Methyl- oder ein Ethylradikal ist

2. Verfahren zum Erhalt neuer Perfluoroalkylen-Verbindungen gemäß Patentanspruch (1), gekennzeichnet dadurch, daß man ein Epihalohydrin mit einer Perfluoro- oder einer Polyfluoroalkylen-Verbindung mit einer von der Fluorokette abgesetzten tertiären Aminofunktion, reagieren läßt, und die folgender allgemeingültiger Formel entspricht: 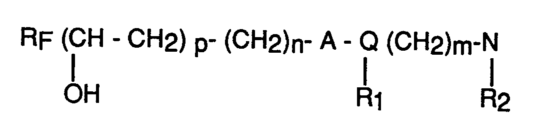 Die Bedeutungen sind in Patentanspruch (1) definiert.

3. Verfahren zum Erhalt neuer Pertluoroalkylen-Verbindungen gemäß Patentanspruch (2), gekennzeichnet dadurch, daß das Epihalohydrin das Epichlorhydrin von Glyzerin ist.

4. Neue Fluoroverbindungen gemäß Patentanspruch (1), gekennzeichnet dadurch, daß man sie im allgemeinen als Fluoroalkylenmittel in öl- und/oder wasser- und/oder alkoholabstoßenden Techniken und im besonderen bei der Fluoroalkylbildung von Protein-, Polyol- oder Polysaccharid-Derivaten einsetzt.

5. Neue Fluoroverbindungen gemäß Patentanspruch (1), gekennzeichnet dadurch, daß man sie als spannungsaktive, benetzende, grenzflächenaktive Mittel und als Komponente für Bakterizide oder Korrosions-Hemmstoffe verwendet, oder daß man sie bei der Herstellung von Löschmitteln einsetzt.
